(19) Europäisches Patentamt
European Patent Office
Office européen des brevets

(11) **EP 3 725 222 B1**

(12) **EUROPÄISCHE PATENTSCHRIFT**

(45) Veröffentlichungstag und Bekanntmachung des Hinweises auf die Patenterteilung:
**12.07.2023 Patentblatt 2023/28**

(21) Anmeldenummer: **20000151.9**

(22) Anmeldetag: **14.04.2020**

(51) Internationale Patentklassifikation (IPC):
**A61B 5/0536** (2021.01) **A61M 16/00** (2006.01)
**A61B 5/00** (2006.01) **A61B 5/085** (2006.01)
**A61B 5/08** (2006.01)

(52) Gemeinsame Patentklassifikation (CPC):
(C-Sets verfügbar)
**A61B 5/0536; A61B 5/0803; A61B 5/0809;**
**A61B 5/6803; A61B 5/743; A61M 16/024;**
A61B 5/0816; A61M 16/0066; A61M 2016/0027;
A61M 2016/0033; A61M 2202/0208;
A61M 2205/3569; A61M 2205/3592;
A61M 2205/502; A61M 2209/088; (Forts.)

(54) **SYSTEM UND BEATMUNGSGERÄT ZUR ECHTZEIT-BESTIMMUNG EINER LOKALEN BEANSPRUCHUNG EINER LUNGE WÄHREND DER KÜNSTLICHEN BEATMUNG**

SYSTEM AND VENTILATOR FOR REAL-TIME DETERMINATION OF LOCAL STRESS OF A LUNG DURING ARTIFICIAL RESPIRATION

SYSTÈME ET APPAREIL RESPIRATOIRE DE DÉTERMINATION EN TEMPS RÉEL D'UNE CONTRAINTE LOCALE D'UN POUMON PENDANT LA RESPIRATION ARTIFICIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2019 DE 102019109932**

(43) Veröffentlichungstag der Anmeldung:
**21.10.2020 Patentblatt 2020/43**

(73) Patentinhaber: **Löwenstein Medical Technology S.A.**
**2557 Luxembourg (LU)**

(72) Erfinder:
• **Kremeier, Peter**
**76149 Karlsruhe (DE)**
• **Tusman, Gerado**
**7600 Mar del Plata (AR)**
• **Pulletz, Sven**
**49090 Osnabrück (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
**DE-A1-102013 203 177 DE-B3-102005 022 896**
**DE-B3-102015 006 902**

• ATSUKO SHONO ET AL: "Clinical implication of monitoring regional ventilation using electrical impedance tomography", JOURNAL OF INTENSIVE CARE, BIOMED CENTRAL LTD, LONDON, UK, Bd. 7, Nr. 1, 18. Januar 2019 (2019-01-18) , Seiten 1-10, XP021270841, DOI: 10.1186/S40560-019-0358-4

(52) Gemeinsame Patentklassifikation (CPC): (Forts.)
A61M 2230/04; A61M 2230/10; A61M 2230/14;
A61M 2230/202; A61M 2230/205; A61M 2230/60;
A61M 2230/65

C-Sets
A61M 2230/65, A61M 2230/005

(52) Gemeinsame Patentklassifikation (CPC): (Forts.)
A61M 2230/04; A61M 2230/10; A61M 2230/14;
A61M 2230/205; A61M 2230/60;

**Beschreibung**

Die vorliegende Erfindung betrifft ein System zur Echtzeit-Bestimmung einer lokalen Beanspruchung einer Lunge während einer künstlichen Beatmung. Ferner betrifft die Erfindung ein System zur automatisierten Einstellung eines durch ein Beatmungsgerät vorgegebenen Wertes, insbesondere eines Drucks, vorzugsweise eines positiven endexspiratorischen Drucks (PEEP), umfassend ein vorgenanntes System zur Echtzeit-Bestimmung einer lokalen Beanspruchung einer Lunge während einer künstlichen Beatmung, sowie weiterhin ein Beatmungsgerät umfassend ein vorgenanntes System zur automatisierten Einstellung eines durch ein Beatmungsgerät vorgegebenen Wertes. Ein Verfahren zur Bestimmung einer lokalen Beanspruchung einer Lunge während einer künstlichen Beatmung, sowie ein Verfahren zur automatisierten Einstellung eines durch ein Beatmungsgerät vorgegebenen Wertes sind nicht erfindungsgemäß und dienen der Veranschaulichung. Weiterhin betrifft die Erfindung ein entsprechendes Beatmungsgerät.

Bei maschineller Beatmung von Patienten mittels eines Beatmungsgerätes wird dem Patienten Atemgas mit Überdruck zugeführt. Deswegen ist bei der Beatmung der Atemwegsdruck bzw. alveoläre Druck zumindest während der Inspirationsphase größer als der Druck im die Lungenbläschen bzw. Alveolen umgebenden Pleuraspalt. Wird in der Exspirationsphase die Druckbeaufschlagung des Atemwegs durch die Beatmungseinrichtung wieder zurückgefahren, so hat dies die Folge, dass das Lungengewebe sich entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Exspirationsphase derart ungünstig einstellen, dass es zu einem Kollaps von Teilen der Alveolen kommt. Dann muss der kollabierte Teil des Lungenvolumens im nachfolgenden Atmungszyklus erst wieder entfaltet werden. Die funktionelle Residualkapazität der Lunge wird dadurch negativ beeinträchtigt, so dass die Sauerstoffsättigung abnimmt, und auch das Lungengewebe kann Schaden nehmen.

Um einen Kollaps von Alveolen am Ende der Exspirationsphase vorzubeugen, wird bei der maschinellen Überdruckbeatmung regelmäßig ein sogenannter positiver end-exspiratorischer Druck - im Folgenden kurz PEEP genannt - eingestellt.

Bei künstlicher Beatmung mit PEEP beaufschlagt die Beatmungseinrichtung den Atemweg dauerhaft - also sowohl während der Inspirationsphase als auch während der Exspirationsphase - mit zumindest einem vorbestimmten Überdruck, dem PEEP. Der PEEP liegt also nach dem Ende der Exspirationsphase noch an.

Es gilt, den PEEP möglichst so einzustellen, dass während der Exspirationsphase der alveoläre Druck nicht, oder jedenfalls nur so weit, unterhalb des Druckes im Pleuraspalt liegt, dass das alveoläre Gewebe unter der Wirkung des Drucks im Pleuraspalt nicht kollabiert. Allerdings kann ein zu hoher Wert des PEEP negative Folgen haben, insbesondere während der Inspirationsphase. Denn das Lungengewebe kann bei sehr hohen Atemwegsdrücken während der Inspirationsphase überdehnt werden. Es kommt lokal zu einer zu großen Beanspruchung der Lunge.

Es ist daher erstrebenswert, die Beanspruchung der Lunge möglichst genau beobachten zu können, um Rückschlüsse auf einen gegebenenfalls zu hoch oder zu niedrig eingestellten PEEP - bzw. andere die künstliche Beatmung charakterisierende Parameter - zu ziehen.

Im Stand der Technik ist es üblich, die Belastung bzw. Beanspruchung der Lunge als sogenannten "strain" anzugeben, indem das Tidalvolumen auf die funktionale Residualkapazität der Lunge bezogen wird. Die Beanspruchung der Lunge ("strain"; STR) lässt sich somit über die folgende Gleichung bestimmen:

$$STR = Tidalvolumen / FRC$$

STR: "strain" (Beanspruchung der Lunge)
FRC: funktionale Residualkapazität

Problematisch bei einer solchen Bestimmung der Beanspruchung der Lunge ist jedoch zum einen, dass die funktionale Residualkapazität nur schwer bestimmbar ist, in besonderem Maße während einer künstlichen Beatmung eines Patienten. Zum anderen liefert eine solche, oben genannte Bestimmung der Beanspruchung der Lunge nur einen globalen Wert für die Beanspruchung der Lunge. Es ist keine örtliche Auflösung dahingehend möglich, welche Bereiche der Lunge mehr und welche weniger belastet sind. Ein Rückschluss über lokal überdehnte Lungenbereiche oder lokal kollabierte Abschnitte der Lunge ist nicht möglich.

An dieser Stelle kann die Elektro-Impedanz-Tomographie - im Folgenden kurz EIT genannt - Abhilfe leisten. EIT ist ein bildgebendes Verfahren, mittels welchem auf nicht-invasive Weise Rückschlüsse auf den Zustand der Lunge eines Patienten gezogen werden können. Hierzu wird ausgenutzt, dass die durch Atembewegungen des Patienten beeinflusste Änderung eines mittels EIT gewonnenen Impedanzsignals in Bereichen, in denen die Lunge noch nicht kollabiert ist, größer ist als in Bereichen mit kollabierten Alveolen.

Durch EIT steht ein Verfahren zur Verfügung, das erlaubt, in Echtzeit Information über den Zustand der Lunge, insbesondere über das Öffnen und Schließenvon Alveolen oder eine Überdehnung von Alveolen, zu erhalten. Die EIT kann direkt am Bett des Patienten durchgeführt werden und liefert räumlich differenzierte Informationen hinsichtlich unterschiedlicher Bereiche der Lunge. Zu Details der EIT wird auch auf das Dokument DE 10 2013 203 177 A1 verwiesen.

Es ist nun wünschenswert, dass auf möglichst einfache Art und Weise, Rückschlüsse auf die lokale Beanspruchung einer Lunge während einer künstlichen Beatmung geschlossen werden können, um idealerweise auch Handlungsweisungen auf die für die künstliche Beatmung einzustellenden Parameter abzuleiten.

Demnach liegt der vorlegenden Erfindung die Aufgabe zugrunde, ein System zur Echtzeit-Bestimmung einer lokalen Beanspruchung einer Lunge während einer künstlichen Beatmung bereitzustellen, das auf nicht-invasive, schnelle und verlässliche Weise die lokale Beanspruchung der Lunge darstellen kann.

Eine weitere Aufgabe ist es, ein System zur automatisierten Einstellung eines durch ein Beatmungsgerät vorgegebenen Wertes bereitzustellen, das auf schnelle und verlässliche Weise die künstliche Beatmung der Lunge kontrollieren kann.

Eine weitere Aufgabe ist es, ein Beatmungsgerät bereitzustellen, das auf verlässliche Weise eine künstliche Beatmung der Lunge durchführen kann, wobei die Gefahr einer Überdehnung oder eines Kollapses von Bereichen der Lunge reduziert ist.

Eine weitere Aufgabe ist es, ein Verfahren zur Bestimmung einer lokalen Beanspruchung einer Lunge während einer künstlichen Beatmung bereitzustellen, mittels dessen auf nicht-invasive, schnelle und verlässliche Weise die lokale Beanspruchung der Lunge bestimmt werden kann. Eine weitere Aufgabe ist es, ein Verfahren zur automatisierten Einstellung eines durch ein Beatmungsgerät vorgegebenen Wertes bereitzustellen, mittels dessen auf verlässliche Weise die künstliche Beatmung der Lunge kontrolliert werden kann.

Gelöst werden die genannten Aufgaben durch ein System zur Echtzeit-Bestimmung einer lokalen Beanspruchung einer Lunge während einer künstlichen Beatmung nach Anspruch 1, durch ein System zur automatisierten Einstellung eines durch ein Beatmungsgerät vorgegebenen Wertes nach Anspruch 4 sowie durch ein Beatmungsgerät nach Anspruch **5**. Ein Verfahren zur Bestimmung einer lokalen Beanspruchung einer Lunge während einer künstlichen Beatmung, sowie ein Verfahren zur automatisierten Einstellung eines durch ein Beatmungsgerät vorgegebenen Wertes sind nicht erfindungsgemäß und dienen der Veranschaulichung.

Die Unteransprüche betreffen verschiedene voneinander unabhängige, vorteilhafte Weiterbildungen der vorliegenden Erfindung.

Im Einzelnen wird nach einem ersten Aspekt der Erfindung ein System zur Echtzeit-Bestimmung einer lokalen Beanspruchung einer Lunge während einer künstlichen Beatmung vorgeschlagen. Das System umfasst ein Beatmungsgerät, wobei das Beatmungsgerät Teil des Systems ist; wobei eine Einrichtung zur Elektro-Impedanz-Tomographie (EIT) vorgesehen ist, die dazu eingerichtet ist, eine elektrische Impedanzverteilung entlang wenigstens eines zweidimensionalen Schnitts durch einen menschlichen Thorax zu erfassen; wobei eine Einrichtung zur Zuordnung der erfassten elektrischen Impedanzverteilung vorgesehen ist, die dazu eingerichtet ist, die erfasste elektrische Impedanzverteilung zu verschiedenen Zeitpunkten während der künstlichen Beatmung in eine Vielzahl von EIT-Pixeln zu unterteilen und einen spezifischen Wert der elektrischen Impedanz zu einem spezifischen Zeitpunkt einem spezifischen EIT-Pixel zuzuweisen, wobei eine Einrichtung zur Bestimmung eines lokalen Beanspruchungswertes vorgesehen ist, die dazu eingerichtet ist; zumindest eine end-inspiratorische elektrische Impedanz (ZINSP) des spezifischen EIT-Pixels sowie eine end-exspiratorische elektrische Impedanz (ZEXSP) des spezifischen EIT-Pixels zu bestimmen und einen lokalen Tidalvolumenreferenzwert ($\Delta Z$) des spezifischen EIT-Pixels als Differenz aus der end-inspiratorischen elektrischen Impedanz (ZINSP) und der end-exspiratorischen elektrischen Impedanz (ZEXSP) durch Subtraktion der end-exspiratorischen elektrischen Impedanz (ZEXSP) von der end-inspiratorischen elektrischen Impedanz (ZINSP) zu bilden, dadurch gekennzeichnet, dass die Einrichtung dazu eingerichtet ist; einen vorläufigen Beanspruchungswert (STRVORL) des spezifischen EIT-Pixels durch Dividieren des lokalen Tidalvolumenreferenzwertes ($\Delta Z$) durch die end-exspiratorische elektrische Impedanz (ZEXSP) zu bilden und einen relativen Beanspruchungswert (STRRELATIV) des spezifischen EIT-Pixels durch Normierung des vorläufigen Beanspruchungswert (STRVORL) auf einen Referenz-Beanspruchungswert (STRREF) zu bilden.

Wesentlich an der vorliegenden Erfindung ist, dass mithilfe des beschriebenen Systems eine örtlich aufgelöste Beobachtung der Lunge hinsichtlich einer eventuellen Überdehnung vorgenommen werden. Diese Beobachtung kann darüber hinaus in Echtzeit während einer künstlichen Beatmung vorgenommen werden. Dies kann erfolgen, ohne dass aufwendige Eingriffe zu Lasten des Patienten erforderlich wären, da die EIT ein nicht-invasives, bildgebendes Verfahren ist.

Dadurch dass die erfassten Impedanzwerte als Referenzwerte für ein in einem spezifischen EIT-Pixel vorhandenen Volumen dienen können, kann auf einzelne Bereiche der Lunge aufgelöst eine lokale Bestimmung eines analogen Wertes für das Tidalvolumen im spezifischen EIT-Pixel vorgenommen werden. Wenn dieser, das Tidalvolumen repräsentierende Referenz-Impedanzwert nun auf den Referenz-Impedanzwert, welcher das in demselben EIT-Pixel verbleibende Volumen repräsentiert, bezogen wird, kann ein Wert erreicht werden, welcher die Beanspruchung in diesem spezifischen Bereich der Lunge wiedergibt. So kann anstelle der globalen Bestimmung der Beanspruchung bzw. des "strain" gemäß Stand der Technik nun eine lokale Auflösung erreicht werden und die lokale Beanspruchung bzw. der lokale "strain" bestimmt werden.

Diese lokale Bestimmung erfolgt dementsprechend für ein spezifisches EIT-Pixel nach der folgenden Gleichung:

$$STR_{VORL} = \Delta Z / Z_{EXSP}$$

$STR_{VORL}$: vorläufiger Beanspruchungswert
$\Delta Z$: lokaler Tidalvolumenreferenzwert
$Z_{EXSP}$: end-exspiratorische elektrische Impedanz

Zur Bestimmung des lokalen Tidalvolumenreferenzwertes ($\Delta Z$) des spezifischen EIT-Pixels wird erfindungsgemäß eine Subtraktion der end-exspiratorischen elektrischen Impedanz ($Z_{EXSP}$) von der end-inspiratorischen elektrischen Impedanz ($Z_{INSP}$) vorgenommen werden.

Mittels des vorliegenden Systems wird die end-inspiratorische elektrische Impedanz ($Z_{INSP}$) sowie die end-exspiratorische elektrische Impedanz ($Z_{EXSP}$) bestimmt. Daraus wird die Differenz als Repräsentant für das lokale Tidalvolumen in dem konkreten Lungenbereich bestimmt. Schließlich wird durch den Bezug dieser Referenz erneut auf die - das in dem konkreten Bereich verbleibende Volumen repräsentierende - end-exspiratorische elektrische Impedanz ($Z_{EXSP}$) ein die Beanspruchung des Lungenbereichs repräsentierender Wert erreicht.

Die end-exspiratorische elektrische Impedanz ($Z_{EXSP}$) entspricht dabei dem Endwert der elektrischen Impedanz zum Ende der Exspiration in dem spezifischen EIT-Pixel. Sie kann auch kurz EELI genannt werden.

Dieser, die Beanspruchung des konkreten Lungenbereiches repräsentierende Wert wird vorliegend "vorläufiger Beanspruchungswert" genannt. Abschließend wird mittels des vorliegenden Systems dieser vorläufige Beanspruchungswert noch normiert. Eine solche Normierung meint, dass ein Quotient aus dem vorläufigen Beanspruchungswert und einem Referenz-Beanspruchungswert ($STR_{REF}$) gebildet wird. Auf diese Weise wird schließlich ein relativer Beanspruchungswert erzielt, der die lokale Beanspruchung der Lunge repräsentiert.

Durch die Normierung wird eine Vergleichbarkeit erzielt, indem der spezifische, in Echtzeit bestimmte, vorläufige Beanspruchungswert noch auf einen Referenz-Beanspruchungswert bezogen wird, der den Zustand der "offenen Lunge" repräsentiert. Unter "offener Lunge" ist dabei gemeint, dass die Lunge weder kollabiert noch überdehnt ist. Es wird also der konkret bestimmte, vorläufige Beanspruchungswert dadurch normalisiert, dass er auf einen die Lunge repräsentierenden Wert in einem annähernden Normalzustand bezogen wird. Auf diese Weise wird ein relativer Beanspruchungswert erzielt, der die lokale Beanspruchung der Lunge repräsentiert.

Nach einer ersten vorteilhaften Ausführungsform kann es sich bei dem Referenz-Beanspruchungswert ($STR_{REF}$) um einen Quotienten aus dem lokalen Tidalvolumenreferenzwert ($\Delta Z$) und der end-exspiratorischen elektrischen Impedanz ($Z_{EXSP}$) des spezifischen EIT-Pixels bei einem vorgegebenen positiven endexspiratorischen Druck (PEEP) handeln. Dementsprechend kann die Einrichtung zur Bestimmung eines lokalen Beanspruchungswertes für die Bildung des relativen Beanspruchungswert ($STR_{RELATIV}$) des spezifischen EIT-Pixels derart ausgebildet sein, dass der vorläufige Beanspruchungswert ($STR_{VORL}$) auf einen Quotienten aus dem lokalen Tidalvolumenreferenzwert ($\Delta Z$) und der end-exspiratorischen elektrischen Impedanz ($Z_{EXSP}$) des spezifischen EIT-Pixels bei einem vorgegebenen positiven endexspiratorischen Druck (PEEP) normiert werden kann. Bei diesem vorgegebenen positiven endexspiratorischen Druck (PEEP) kann es sich um einen sogenannten "open lung PEEP" oder kurz "OL-PEEP" handeln. Bei diesein Wert für den vorgegebenen positiven endexspiratorischen Druck in Form eines "open lung PEEP" bzw. "OL-PEEP" kann ein end-exspiratorischer Kollaps der Lunge vermieden werden. Dieser Ausführungsform liegt also die Überlegung zugrunde, dass bei einer künstlichen Beatmung mit einem solchen vorgegebenen PEEP die Verhältnisse in der Lunge derart sind, dass die Gefahr eines Kollapses von Lungenbereichen aber auch einer Überdehnung von Lungenbereichen minimal ist. Durch die Normierung auf diesen sogenannten "Normalzustand" kann eine besonders gute Online-Beobachtung in Echtzeit der Belastung einzelner Lungenbereiche erzielt werden. Auf diese Weise kann der Einfluss des PEEP, oder auch anderer die künstliche Beatmung des Patienten steuernder Parameter, auf die lokale Belastung der Lunge beobachtet und demnach über die Steuerung der Parameter kontrolliert werden.

Der relative Beanspruchungswert ($STR_{RELATIV}$) eines spezifischen EIT-Pixels kann demnach über die folgende Gleichung bestimmt werden:

$$STR_{RELATIV} = STR_{VORL} / (\Delta Z_{OL\text{-}PEEP} / Z_{EXSP, OL\text{-}PEEP})$$

$$= (\Delta Z / Z_{EXSP}) / (\Delta Z_{OL\text{-}PEEP} / Z_{EXSP, OL\text{-}PEEP})$$

$STR_{RELATIV}$:     relativer Beanspruchungswert

$STR_{VORL}$:  vorläufiger Beanspruchungswert

$\Delta Z_{OL\text{-}PEEP}$:  Tidalvolumenreferenzwert bei OL-PEEP

$Z_{EXSP,\,OL\text{-}PEEP}$:  end-exspiratorische elektrische Impedanz bei OL-PEEP

$\Delta Z$:  lokaler Tidalvolumenreferenzwert

$Z_{EXSP}$:  end-exspiratorische elektrische Impedanz

Als Referenz für die beschriebene Normierung kann der Quotient aus lokalem Tidalvolumenreferenzwert ($\Delta Z$) und der end-exspiratorischen elektrischen Impedanz ($Z_{EXSP}$) beispielsweise bei einem positiven endexspiratorischen Druck in Höhe von 15 $cmH_2O$ verwendet werden. Alternativ kann auch ein anderer positiver endexspiratorischer Druck als Referenzwert dienen.

Dabei können verschiedene (als solche nicht unter die Ansprüche fallende) Verfahren angewandt werden, um vorab den "OL-PEEP" zu bestimmen und schließlich den zuvor beschriebenen Schritt der Normierung erfolgreich durchzuführen:

- So kann als "OL-PEEP" der Wert verwendet werden, bei dem der transmurale Druck größer als 0 ist.
- Auch kann als "OL-PEEP" der Wert verwendet werden, bei dem der Pesophagus-Druck größer als 0 ist.
- Auch kann mittels EIT ein "OL-PEEP" ermittelt werden, indem eine Druckrampe gefahren und der Druck bestimmt wird, bei dem sich in den abhängigen Lungenpixeln eine plötzliche Zunahme der Impedanz gibt. Dies deutet den Öffnungsdruck der Pixelregion an.
- Auch mittels der sogenannten "best dynamic compliance Technik" kann ein "OL-PEEP" ermittelt werden. Insofern wird auf das Paper von Suarez-Sipmann verwiesen (Crit Care Med 2007 Vol. 35, No. 1, S. 214 - 221).
- Auch mittels EIT gemäß Costa kann ein "OL-PEEP" bestimmt werden. Insofern wird auf das Paper von Cosat verwiesen (Intensive Care Med; DOI 10.1007/s00134-009-1447-y).
- Durch direkte Messung des exspiratorischen Lungenvolumens, ist ebenfalls eine Ermittlung eines Referenzwertes zwecks Normierung auf ein annähernd normales Lungenvolumen (welches bei gesunder Lungen und einem PEEP in der Höhe von 0 der funktionalen Residualkapazität - FRC - entspricht) möglich.

Nach einem weiteren Aspekt der Lehre wird ein System zur automatisierten Einstellung eines durch ein Beatmungsgerät vorgegebenen Wertes, insbesondere eines Drucks, vorzugsweise eines positiven endexspiratorischen Drucks (PEEP), vorgeschlagen, umfassend ein vorbeschriebenes System, wobei eine Regelungseinrichtung vorgesehen ist, die dazu eingerichtet ist, den durch das Beatmungsgerät vorgegebenen Wert auf Grundlage der gebildeten relativen Beanspruchungswerte ($STR_{RELATIV}$) anzupassen.

Nach einem weiteren Aspekt der Lehre wird ein Beatmungsgerät vorgeschlagen, umfassend ein solches System zur automatisierten Einstellung eines durch ein Beatmungsgerät vorgegebenen Wertes.

Nach einem weiteren nicht unter die Ansprüche fallenden Aspekt der Lehre wird ein Beatmungsgerät zur Ausführung der Verfahren und/oder zur Verwendung in den Systemen vorgeschlagen.

Beispielsweise ist die Erfindung auch dadurch gekennzeichnet, dass die Einrichtung zur Elektro-Impedanz-Tomographie (EIT) mit dem Beatmungsgerät gekoppelt ist oder Teil des Beatmungsgerätes ist.

Durch die Übertragung des zuvor beschriebenen Systems zur Echtzeit-Bestimmung einer lokalen Beanspruchung einer Lunge während einer künstlichen Beatmung beispielsweise auf ein Beatmungsgerät kann die künstliche Beatmung eines Patienten dahingehend verbessert werden, dass die gewonnenen Kenntnisse über die lokale Belastung einzelner Lungenareale dazu eingesetzt werden können, die Beatmungsparameter anzupassen. So kann beispielsweise der PEEP insbesondere dann angepasst werden, wenn festgestellt wird, dass einzelne Lungenareale einer zu hohen Beanspruchung ausgesetzt sind. Dies kann beispielsweise dadurch detektiert werden, dass der relative Beanspruchungswert hoch ist. Durch die Anpassung des PEEP (beispielsweise in Form einer Erhöhung oder Verringerung) oder auch durch die Anpassung anderer Beatmungsparameter kann in Echtzeit die Änderung des relative Beanspruchungswert ($STR_{RELATIV}$) beobachtet werden. Ein anderer einzustellender Beatmungsparameter kann beispielsweise das Tidalvolumen sein. Denn, wenn das an dem Beatmungsgerät vorgesehene Tidalvolumen verringert wird, so wird dadurch auch in der Regel die Überdehnung bzw. der "strain" der Lunge verringert werden können. Es können auch Mittel, beispielsweise eine Regelungseinrichtung, vorgesehen sein, die den PEEP oder andere Werte wie das Tidalvolumen oder andere Werte automatisch anpassen, sodass jederzeit ein gewünschter, beispielsweise besonders geringer, relativer Beanspruchungswert ($STR_{RELATIV}$) vorliegt.

Nach einem weiteren Aspekt der Lehre wird ein (nicht unter die Ansprüche fallendes) Verfahren zur Bestimmung einer lokalen Beanspruchung einer Lunge während einer künstlichen Beatmung vorgeschlagen, insbesondere zur Durchführung in einem System nach einem der Ansprüche 1 bis 2. Dabei werden die folgende Schritte ausgeführt: Erfassen einer elektrischen Impedanzverteilung entlang wenigstens eines zweidimensionalen Schnitts durch einen menschlichen Thorax mittels einer Einrichtung zur Elektro-Impedanz-Tomographie (EIT); Unterteilen der erfassten elektrischen Impedanzverteilung zu verschiedenen Zeitpunkten während der künstlichen Beatmung in eine Vielzahl von EIT-Pixeln; Zuweisen eines spezifischen Wertes der elektrischen Impedanz einem spezifischen EIT-Pixel zu einem spezifischen Zeitpunkt; Bestimmen zumindest einer end-inspiratorischen elektrischen Impedanz ($Z_{INSP}$) des spezifischen EIT-Pixels sowie einer end-exspiratorischen elektrischen Impedanz ($Z_{EXSP}$) des spezifischen EIT-Pixels; Bilden eines lokalen Tidalvolumenreferenzwertes ($\Delta Z$) des spezifischen EIT-Pixels als Differenz aus der end-inspiratorischen elektrischen Impedanz ($Z_{INSP}$) und der end-exspiratorischen elektrischen Impedanz ($Z_{EXSP}$), insbesondere durch Subtraktion der end-exspiratorischen elektrischen Impedanz ($Z_{EXSP}$) von der end-inspiratorischen elektrischen Impedanz ($Z_{INSP}$); Bilden eines vorläufigen Beanspruchungswertes ($STR_{VORL}$) des spezifischen EIT-Pixels durch Dividieren des lokalen Tidalvolumenreferenzwertes ($\Delta Z$) durch die end-exspiratorische elektrische Impedanz ($Z_{EXSP}$); und Bilden eines relativen Beanspruchungswertes ($STR_{RELATIV}$) des spezifischen EIT-Pixels durch Normierung des vorläufigen Beanspruchungswert ($STR_{VORL}$) auf einen Referenz-Beanspruchungswert ($STR_{REF}$).

Nach einer weiteren vorteilhaften Ausführungsform des (nicht unter die Ansprüche fallenden) Verfahrens wird der Referenz-Beanspruchungswert ($STR_{REF}$) als Quotient aus dem lokalen Tidalvolumenreferenzwert ($\Delta Z$) und der end-exspiratorischen elektrischen Impedanz ($Z_{EXSP}$) des spezifischen EIT-Pixels bei einem vorgegebenen positiven endexspiratorischen Druck (PEEP) gebildet.

Die konkreten Auswirkungen und Vorteile dieser Verfahren sind zuvor bereits hinsichtlich des Systems zur Echtzeit-Bestimmung einer lokalen Beanspruchung einer Lunge während einer künstlichen Beatmung beschrieben worden. Insofern wird auf diese Bezug genommen.

Weiter vorzugsweise kann bei dem (nicht unter die Ansprüche fallenden) Verfahren zunächst ein relevantes Lungenareal identifiziert werden. Durch diesen Schritt kann die Effizienz des Verfahrens weiter erhöht werden. So kann durch einen vorgeschalteten Schritt genau der Bereich erfasst werden, bei welchem zu beobachtendes Lungengewebe bei dem Patienten vorliegt. Die EIT kann dementsprechend optimiert werden, da die impedanzmessungen nur hinsichtlich der Areale von Bedeutung sind, in denen auch wirklich Lungengewebe vorhanden ist.

Dazu können beispielsweise zunächst Referenz-Impedanzmessungen vorgenommen werden. So kann beispielsweis die elektrische Impedanz bei einer künstlichen Beatmung unter erhöhtem PEEP bestimmt werden. Bei einem erhöhtem PEEP wird nämlich davon ausgegangen, dass auch unter Umständen kollabiertes Lungengewebe sich erneut mit Luft füllt und entfaltet und dementsprechend auch einen Ausschlag bei der Impedanzmessung zeigt. Alle Bereiche, die bei einer Impedanzmessung unter erhöhtem PEEP demnach eine signifikante, beispielsweise über einem vorgegebenen Schwellenwert liegende, Impedanzdifferenz (etwa in Form der lokalen Tidalvolumenreferenzwertes $\Delta Z$) zeigen, können somit als relevantes Lungenareal identifiziert werden.

Besonders bevorzugt können zur Identifizierung des relevanten Lungenareals auch mehrere Messungen bei verschiedenen PEEP-Werten vorgenommen werden und jeweils bei den unterschiedlichen Messungen die Pixel, welche eine vorhin genannte, signifikante Impedanzdifferenz zeigen, als relevantes Lungenareal gekennzeichnet werden. Denn gerade bei einem niedrigem PEEP kann es zwar zur Belüftung zuvor überdehnter Lungenbläschen, vor allem in den Schwerkraft-unabhängigen oberen Lungenpartien, kommen, während jedoch die zuunterst liegenden Lungenareale weiterhin kollabieren. Diese zuunterst liegenden Lungenareale zeigen beispielsweise erst bei einem höheren PEEP eine Ventilation, die sich in einer relevanten signifikanten Impedanzdifferenz äußert. Demnach sollte eine Vielzahl verschiedener PEEP Werte bei der Identifizierung des relevanten Lungenareals eingestellt werden und die Pixel als Lungenpixel identifiziert werden, die auf wenigstens einer der PEEP-Stufen tidale Ventilation zeigen.

Bei den zuvor beschriebenen Systemen kann dementsprechend auch eine Einrichtung zur Identifizierung eines relevanten Lungenareals vorgesehen sein, die dazu eingerichtet ist, ein relevantes Lungenareal zu identifizieren.

Schließlich wird nach einem weiteren Aspekt der Lehre ein (nicht unter die Ansprüche fallendes) Verfahren zur automatisierten Einstellung eines durch ein Beatmungsgerät vorgegebenen Wertes, insbesondere eines Drucks, vorzugsweise eines positiven endexspiratorischen Drucks (PEEP), vorgeschlagen, wobei die folgenden Schritte ausgeführt werden: Bestimmen einer lokalen Beanspruchung einer Lunge während einer künstlichen Beatmung mittels eines Verfahrens zur Bestimmung einer lokalen Beanspruchung einer Lunge während einer künstlichen Beatmung nach einem der Ansprüche 5 bis 7; und Anpassen des durch das Beatmungsgerät vorgegebenen Wertes auf Grundlage der gebildeten relativen Beanspruchungswerte ($STR_{RELATIV}$).

Die konkreten Auswirkungen und Vorteile dieses Verfahrens sind zuvor bereits hinsichtlich des Systems zur automatisierten Einstellung eines durch ein Beatmungsgerät vorgegebenen Wertes beschrieben worden. Insofern wird auf diese Bezug genommen.

Insbesondere kann der zuvor beschriebene Wert in Form des relativen Beanspruchungswertes ($STR_{RELATIV}$) dazu genutzt werden, dass die Parameter für die künstliche Beatmung dahingehend angepasst werden, bis sich bei einer zu hohen Beanspruchung bzw. "strain", sprich bei einem erhöhten relativen Beanspruchungswert ($STR_{RELAIIV}$), (erneut) ein gewünschter niedriger Wert einstellt. So kann durch das vorgeschlagene Verfahren auf zuverlässige Weise sichergestellt_werden, dass eine Überdehnung, jedoch ebenso ein Kollaps, einzelner Lungenbereiche vermieden wird. Beispielsweise kann der PEEP reduziert werden, falls ein zu hoher, über einem Schwellenwert liegender Wert als relativer Beanspruchungswert gemessen wird. Wesentlich ist dabei jedoch auch, dass bei lediglich geringem eingestellten PEEP bei künstlicher Beatmung ebenfalls eine Überdehnung bzw. der "strain" sicher detektiert werden können.

Durch die vorliegende Erfindung ist es auch möglich, die Überdehnung bzw. den "Strain" für die Gesamtlunge in Werten auszudrücken. Dazu kann als Grundlage die Gesamtheit aller Pixelwerte des Lungenareals dienen. So kann der mittlere Überdehnungswert bzw. der mittlere "Strain" aller Pixel im Lungenareal bestimmt werden, indem die "Strain-Werte" aller Pixel aufsummiert werden und diese Summe durch die Anzahl der Pixel im Lungenareal geteilt wird. So können anhand eines einfachen numerischen Wertes verschiedene Beatmungseinstellungen objektiv miteinander verglichen werden. So kann der PEEP oder aber das Tidalvolumen auf Grundlage dieses einfachen Wertes gezielt eingestellt werden.

Mit der vorliegenden Erfindung kann vorteilhaft das Problem der Identifikation von Lungenbelastungen bei einem niedrigem PEEP gelöst werden. Sobald die Lunge zusammenfällt, wird das verbleibende restliche Lungengewebe reaktiv überdehnt. Je größer der Anteil des Kollapses ist, desto mehr Überdehnung findet statt. Genau diese Überdehnung kann durch die vorliegende Erfindung sichtbar gemacht werden. Der Nutzer des Systems kann animiert werden, als Reaktion mehr PEEP zu applizieren, um das für die Ventilation zur Verfügung stehende Lungenvolumen (EELI) zu erhöhen.

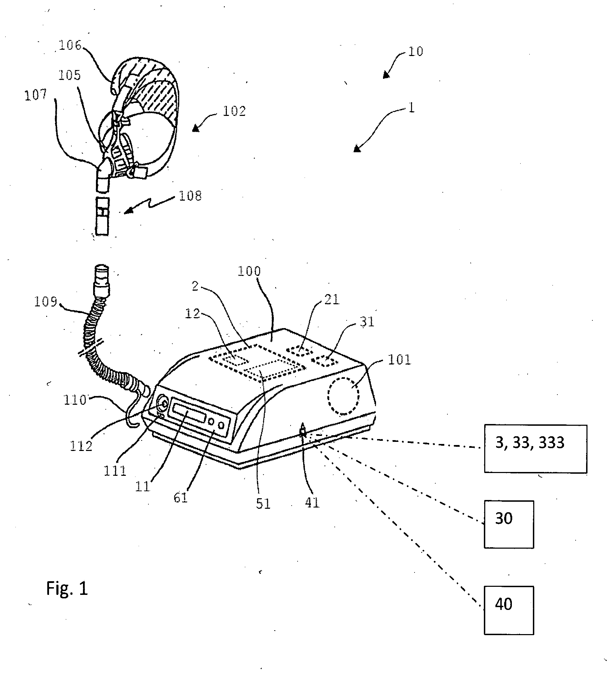

In der Figur 1 ist ein erfindungsgemäßes Beatmungsgerät 1 gezeigt, welches hier ein Heimbeatmungsgerät oder Schlaftherapiegerät oder klinisches Beatmungsgerät eingesetztes Beatmungsgerät 1 sein kann. Das Beatmungsgerät 1 ist zur Durchführung des Verfahrens ausgebildet.

Das Beatmungsgerät 1 ist beispielsweise Teil eines Systems 10 welches eine EIT-Vorrichtung 3 umfasst.

Das Beatmungsgerät 1 umfasst wenigstens eine steuerbare Atemgasquelle 100 und eine programmierbare Steuereinrichtung 21 (Regeleinrichtung) und zumindest eine Sensoreinrichtung 2 zur Bestimmung von Druck und/oder Fluss des Atemgases, wobei die Steuereinrichtung 21 die Atemgasquelle zur Vorgabe eines ersten vorbestimmten Beatmungsmusters 45 (hinsichtlich Druck, Fluss, Volumen, Frequenz) ansteuert. Das Beatmungsgerät umfasst zudem wenigstens zwei weitere beispielsweise räumlich von dem Beatmungsgerät 1 getrennte Sensoreinrichtungen 3, 30, 40, die mit der Steuereinrichtung 21 des Beatmungsgerätes 1 kommunizieren. Ein Sensor 3 ist als EIT (oder EI)-Vorrichtung ausgebildet und weist eine Vielzahl einzelner Sensoreinrichtungen 3, 3', 3" ... auf und jede Sensoreinrichtung ist zur Erzeugung von elektrischen Potenzialen und/oder zur nichtinvasiven Bestimmung der elektrischen Impedanz (EI) eines Körperabschnitts und zur Ermittlung und Übertragung der Sensormesswerte an die Steuereinheit ausgebildet. Die Steuereinrichtung 21 wertet die Sensormesswerte der EI-Sensoreinrichtung 3 aus, zur Bestimmung der momentanen Ventilation 44 oder des "strain" (Beanspruchung) der Lunge während der Beatmung mit einem ersten Beatmungsmuster 45. Die Steuereinrichtung 21 steuert die Atemgasquelle beispielsweise auch zur Vorgabe eines zweiten Beatmungsmusters 55 an, welches hinsichtlich Druck und/oder Fluss und/oder Volumen und/oder Frequenz von dem ersten Beatmungsmuster 45 verschieden ist, wobei die Steuereinrichtung 21 die Sensormesswerte der EI-Sensoreinrichtung 3 auswertet zur Bestimmung der resultierenden Ventilation 54 oder des "strain" der Lunge während der Beatmung mit dem zweiten Beatmungsmuster 55, wobei die Steuereinrichtung 21 die momentanen Ventilation 44 oder den "strain" mit der resultierenden Ventilation 54 oder dem resultierenden "strain" vergleicht zur Feststellung des besser geeigneten Beatmungsmusters. Beispielsweise passt die Steuereinrichtung zumindest einen Druck beispielsweise den PEEP oder ein Beatmungsmuster so an, dass der "strain" reduziert wird.

Beispielsweise wird der "strain" durch eine Erhöhung des EPAP oder des PEEP reduziert.

Das Beatmungsgerät 1 umfasst eine Atemgasquelle 100 mit beispielsweise einer Gebläseeinrichtung und/oder einer Ventileinrichtung 101 zur Erzeugung eines Atemgasstromes für die Beatmung: Zur Steuerung der Atemgasquelle 100 und zur Erfassung und Verarbeitung von Sensordaten ist hier eine Steuereinrichtung 21 vorgesehen. Die Bedienung und Einstellung des Beatmungsgerätes 1 erfolgt über eine Benutzerschnittstelle 61 mit Bedienelementen und einer Anzeigeeinrichtung 11 (Display).

Das Beatmungsgerät 1 weist eine Atemschnittstelle 102 auf, um den Atemgasstrom einem Patienten zur Beatmung zuzuführen. Die hier gezeigte Atemschnittstelle 102 ist eine beispielhaft als Nasalmaske oder Vollmaske ausgebildete Atemmaske 105 oder ein Tubus. Zur Fixierung der Atemmaske 105 ist beispielsweise eine Kopfhaube 106 vorgesehen. Die Atemschnittstelle 102 kann beispielsweise auch als eine Vollgesichtsmaske, als eine Nasenbrille, als ein Tubus oder als eine Larynxmaske ausgestaltet sein.

Zur Verbindung der Atemschnittstelle 102 mit der Atemgasquelle 100 ist zumindest ein Verbindungsschlauch 109 vorgesehen, der über eine Koppeleinrichtung 112 luftführend mit der Atemgasquelle 100 verbunden wird. Über ein Kuppelungselement 107 wird der Verbindungsschlauch 109 mit der Atemschnittstelle 102 verbunden. Zwischen dem Verbindungsschlauch 109 und dem Kuppelungselement 107 ist ein Ausatmungselement 108 angeordnet, welches ein Ventil umfasst oder als ein solches ausgebildet ist. Das Ausatmungselement 108 ist beispielsweise dazu vorgesehen, während der Ausatmung des Benutzers ein Rückatmen in das Beatmungsgerät 1 zu verhindern.

Die Steuereinrichtung 21 ist mit einer nicht näher dargestellten Sensoreinrichtung 2,3,30,40) verbunden, welche einen oder mehrere Sensoren zur Erfassung von Geräteparametern und/oder Patientenparametern und/oder anderer für die Beatmung charakteristischer Grö-ßen aufweist. Die Steuereinrichtung 21 kann als eine zentrale Steuereinrichtung, beispielsweise im dem Beatmungsgerät, ausgeführt sein, die alle Sensorwerte verarbeitet. Die Steuereinrichtung 21 kann in Form mehrerer dezentraler Steuereinrichtungen, beispielsweise eine im dem Beatmungsgerät und (je) eine, die einem (oder den) Sensor(en) zugeordnet ist, ausgeführt sein. für die Beatmung charakteristischer Größen aufweist.

Zum Beispiel umfasst die Steuereinrichtung 21 einen hier nicht näher gezeigten Drucksensor 2 welcher die Druckverhältnisse des Atemgases ermittelt. Dazu ist der Drucksensor, beispielsweise über einen Druckmessschlauch 110 zu der Atemschnittstelle 102, mit dem Atemgas wirkverbunden. Über einen Eingangsstutzen 111 ist der Druckmessschlauch 110 an die Steuereinrichtung 21 angebunden. Zum Beispiel umfasst die Steuereinrichtung 21 auch einen hier nicht näher gezeigten Flusssensor 2, welcher die Flussverhältnisse des Atemgases ermittelt. Des Weiteren dient die Steuereinrichtung 21 hier zur Ansteuerung der Gebläseeinrichtung und/oder der Ventileinrichtung 101. Die Steuereinrichtung 21 stellt einen notwendigen Minimaldruck bereit und kompensiert Druckschwankungen, die durch die Atemtätigkeit des Benutzers bedingt sind. Beispielsweise erfasst die Steuereinrichtung 21 auch den gegenwärtigen Druck in der Atemmaske 105 und regelt die Leistung der Atemgasquelle entsprechend nach, bis ein gewünschter Beatmungsdruck anliegt.

Die zur Einstellung der Atemgasquelle 100 benötigten Geräteparameter sowie die Gerätekonfiguration und/oder Gerätesoftware sind in einer Speichereinrichtung 31 hinterlegt.

Die Steuereinrichtung 21 kann auch den Sauerstoffgehalt im Atemgas regeln, durch entsprechende Ansteuerung der Gebläseeinrichtung und/oder der Ventileinrichtung 101, wobei die Ventileinrichtung zumindest eine Sauerstoffquelle (Druckgasflasche oder Krankenhausleitung) aufweist oder konnektiert. Die Sauerstoffzumischung zu dem Atemgasstrom erfolgt beispielsweise stromabwärts oder stromaufwärts von der Gebläseeinrichtung. In diesem Fall ist die Ventileinrichtung ein Sauerstoffmischventil. Die Sauerstoffzumischung zu dem Atemgasstrom erfolgt beispielsweise durch eine Ventileinheit, die den Atemgasstrom und den Sauerstoffstrom führt und regelt.

Die Steuereinrichtung 21 kann auch zur Erfassung von sensorisch erfassten Patientenparametern ausgebildet sein. Die Steuereinrichtung 21 kann dazu mit Sensoren zur Messung der Atemexkursion, zur Messung einer Sauerstoffversorgung und/oder einer Kohlendioxidversorgung und /oder zur Messung einer EEG-, einer EMG-, einer EOG- oder einer EKG-Aktivität ausgestattet sein.

Das System 10 weist insbesondere wenigstens zwei räumlich von dem Beatmungsgerät 1 getrennte, Sensoreinrichtungen 3,30,40 auf, die mit der Steuereinheit 21 kommunizieren, wobei ein Sensor 3 eine Vielzahl einzelner Sensoreinrichtungen 3, 3', 3'' ... aufweist und jede Sensoreinrichtung zur Erzeugung von elektrischen Potenzialen ausgebildet ist 13 zur nichtinvasiven Bestimmung der elektrischen Impedanz EI eines Körperabschnitts. Die Sensoreinrichtungen 3 sind beispielsweise als Klebepflaster ausgeführt, mit einer der Haut abgewandten Au-ßenschicht und einer der Haut zugewandten adhäsiven Schicht, oder als Gurt zusammengefasst, der die Sensoreinrichtungen haltert.

Das Beatmungsgerät umfasst beispielhaft auch zumindest eine weitere Sensoreinrichtung 30, die zur nichtinvasiven Bestimmung eines Kohlenstoffdioxid (CO2)-Wertes 30 ausgebildet ist. Die Sensoreinrichtung 30 ist beispielsweise als Klemmsensor oder als Klebepflaster ausgeführt mit einer der Haut abgewandten Außenschicht und einer der Haut zugewandten adhäsiven Schicht. Die Beatmungsvorrichtung 10 umfasst zumindest eine weitere Sensoreinrichtung 40, die zur nichtinvasiven Bestimmung eines Sauerstoff (O2)-Wertes 40 ausgebildet ist. Die Sensoreinrichtung 30 ist beispielsweise als Klemmsensor oder als Klebepflaster ausgeführt mit einer der Haut abgewandten Außenschicht und einer der Haut zugewandten adhäsiven Schicht. Der (CO2)-Werte 30 und/oder der (O2)-Werte (40 können erfindungsgemäß invasiv oder nicht-invasiv im Gewebe des Patienten bestimmt werden und/oder nicht-invasiv im Atemgasstrom.

Die Sensoren 3,30,40 umfassen beispielsweise Energie- und Sendemittelmittel zur Ermittlung und kabellosen Übertragung der Sensormesswerte. Die Sensoren 3,30,40 umfassen alternativ beispielsweise Kommunikationsmittel 35, 45 zur Übertragung der Sensormesswerte an die Steuerung 21.

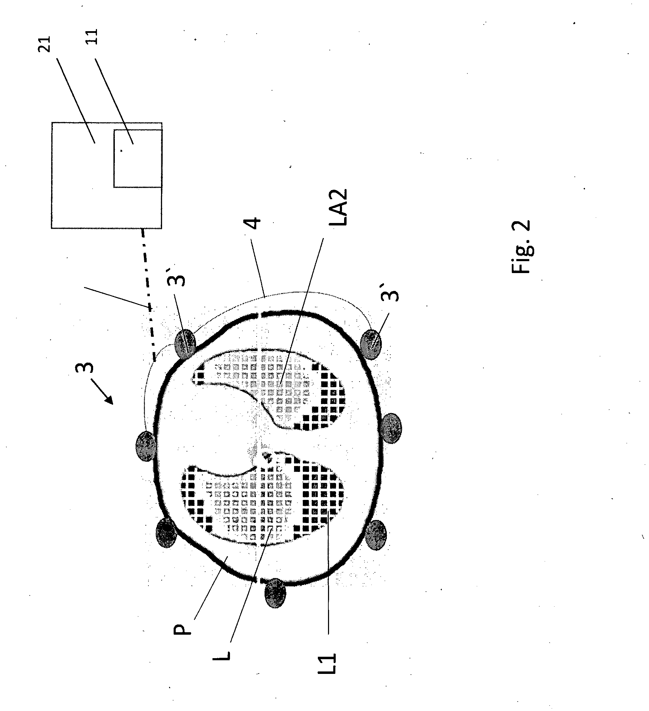

Fig. 2 zeigt eine schematische Darstellung der EIT-Messung unter Beatmung.

Das Beatmungsgerät umfasst beispielhaft auch zumindest eine weitere Sensoreinrichtung 3 zur Durchführung der EIT-Messung.

Die Sensoreinrichtung weist beispielsweise 32 hochauflösende Sensoren 3,3'... in einem textilen Gürtel, nicht dargestellt, auf. Ein, nicht dargestellter, integrierter Lagesensor bestimmt die Position des Patienten. Die Sensoren sind durch Kommunikations- oder Leitungsmittel 4 miteinander verbunden 4 und so angeordnet, dass sie Signale nacheinander aus allen Richtungen empfangen können. Die Steuereinheit 21 kann die Sensormesswerte der EIT-Messung 3 daher räumlich zuordnen.. Damit können die lokalen Gewebswiderstände aus unterschiedlichen Richtungen ermittelt und dann in bewegte Bilder umgerechnet werden. Die Steuereinheit 21 verarbeitet die Sensormesswerte der EIT-Messung 3 derart, dass für einzelne Lungenabschnitte LA1, LA2 eine spezifische elektrische Impedanz (EI) ermittelt wird. Die Sensoreinrichtung 3 ist über kabelgebundene oder drahtlose Kommunikationsmittel 35 mit der Steuereinheit 21 verbunden. Die Steuereinheit 21 speichert 11 und verarbeitet die Sensormesswerte zur Verwendung durch das Beatmungsgerät. Der Speichereinrichtung 31 kann Bestandteil der Steuereinheit 21 sein oder ein separater Baustein.

Fig. 2 zeigt auch eine schematische Darstellung der Ergebnisse der EIT-Messung auf dem Display des Beatmungsgerätes. Die Steuereinheit 21 verarbeitet die Sensormesswerte zur Verwendung durch das Beatmungsgerät derart, dass beispielsweise auf dem Display des Beatmungsgerätes 10 eine Visualisierung des Patiententhorax P mit den beiden Lungenflügeln L dargestellt wird. Die Steuereinheit 21 verarbeitet die Sensormesswerte der EIT-Messung 3 derart, dass für einzelne Lungenabschnitte LA1, LA2 eine spezifische elektrische Impedanz (EI) der Lunge L ermittelt wird. Die spezifische elektrische Impedanz (EI) der Lungenabschnitte LA wird für die Darstellung am Display derart aufbereitet, dass Lungenabschnitte mit einer hohen elektrischen Impedanz L2 graphisch anders dargestellt werden, als solche Lungenabschnitte mit einer niedrigen elektrischen Impedanz L1. Die Steuereinheit 21 verarbeitet die Sensormesswerte der EIT-Messung 3 zeitlich derart, dass für einzelne Lungenabschnitte LA1, LA2 eine spezifische elektrische Impedanz EI der Lunge L atemzugweise ermittelt wird und für die Darstellung am Display aufbereitet wird.

Die Sensoreinrichtung 3 ist (zusammen mit der Steuerung und dem Speicher) auch eingerichtet und ausgebildet, beispielsweise ein EIT-Summensignal 44' zu bilden, welches ein Maß für die Ventilation der Lunge bzw. von Lungenabschnitten ist. Die Sensoreinrichtung 3 ist auch (zusammen mit der Steuerung und dem Speicher) eingerichtet und ausgebildet, eine Häufigkeit der EIT-Änderungsfrequenz 44" zu ermitteln. Die Sensoreinrichtung 3 kann somit zusammenfassend die momentane Ventilation 44 ermitteln.

Im Sinne der Erfindung wird unter dem EIT-Summensignal 44' verstanden, dass die Impedanz- oder Impedanzänderungsverteilung in der durch die Sensoren bestimmten Schnittebene oder dem durch die Sensoren umspannten Volumen nicht zwingend für die Durchführung des Verfahrens bildlich beispielsweise auf einem Display dargestellt werden muss, zumindest aber die berechneten Ergebniswerte der Impedanzverteilung oder Impedanzänderungsverteilung (für die Durchführung des Verfahrens in der erfindungsgemäßen Vorrichtung vorliegen.

Bei einer Summation sämtlicher Impedanzwerte der Sensoren ergibt sich demnach bei der Inspiration ein geändertes Summensignal im Vergleich zu dem Summensignal bei der Exspiration. Der Summenwert stellt demnach ein adäquates Maß dar, um die Ventilation der Lunge zu bestimmen.

Die EIT-Änderungsfrequenz 44" lässt sich aus der atemphasenabhängigen Änderung der Impedanzwerte der Sensoren bestimmen. Die Impedanzwerte der Sensoren schwanken atemphasenabhängig mit der Inspiration und der Exspiration. Die EIT-Änderungsfrequenz 44" stellt demnach ein alternatives oder ergänzendes Maß dar, um die (Frequenz der) Ventilation der Lunge zu bestimmen.

Beispielsweise sind die Sensoren in einen textilen und atmungsaktiven und dehnbaren Elektrodengürtel, zusammen mit einem Lagesensor, integriert. Der Sensor 3 und die Steuereinheit sind eingerichtet eine Bildrate von beispielsweise bis zu 50 pro Sekunde zu erzeugen. Dabei werden Lungenbereiche LA visualisiert. Erfindungsgemäß sind patientenbezogene Eingaben möglich, wie Körpergröße, Körpergewicht, Geschlecht, Brustkorbumfang.

Die so ermittelten und aufbereiteten EIT-Daten ermöglichen eine zeitlich und räumlich aufgelöste differenzierte Analyse und Darstellung der elektrischen Impedanz EI der Lunge.

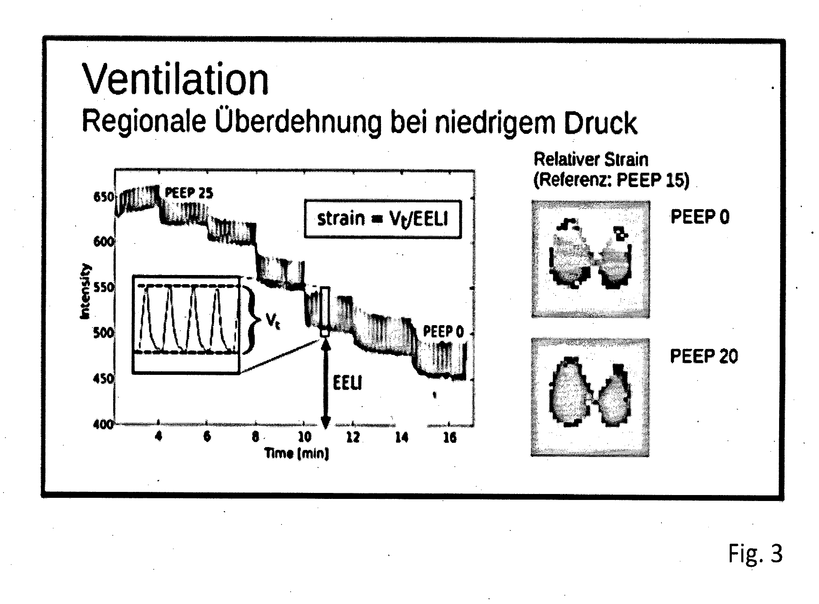

Fig. 3 zeigt die Ermittlung der lokalen Lungenüberdehnung auf Basis der Messung atembedingter Verformungen bei niedrigem PEEP

Alle Berechnungen wurden innerhalb der individuellen Lungen Region of Interest (ROI) durchgeführt, die gebildet wurde von den Pixeln, deren Amplitude mehr als x% der Amplitude des Pixels mit der maximalen atembedingten Impedanzänderung bei PEEP 30 betrug. Erfindungsgemäß kann die Beanspruchung (strain) der gesamten Lungen, die die atemzugsbedingte Verformung des Lungengewebes hervorruft, wie folgt abgeschätzt werden:

$$strain = VT/FRC$$

Die Berechnung der lokalen Beanspruchung (strain) dieser neuartigen EIT-basierten Methode (welche als solche nicht unter die Ansprüche fällt) erfolgt auf Pixelebene, indem das Tidalvolumen eines jeden Pixels aus seiner jeweiligen Impedanzänderung (delta Z) während des Atemzyklus bestimmt und dieses sodann ins Verhältnis zu seinem jeweiligen end-exspiratorischen Volumen, das seinem impedanzwert zum Ende der Exspiration (EELI) entspricht, gesetzt wurde. Die entsprechende Formel lautet daher:

$$Strain = Zend\text{-}expiration - Zend\text{-}inspiration / EELI = Delta\ Z / EELI$$

Zend-exspiration, Zend-inspiration und EELI wurden für jedes Pixel und jeden Atemzug ermittelt, daraus der respektive Mittelwert über alle gemessenen Atemzüge einer jeweiligen PEEP-Stufe berechnet und daraus dann der strain eines jeden Pixels berechnet.

Anschließend wurde der Pixel-strain auf jeder PEEP-Stufe ins Verhältnis gesetzt zum individuellen Pixel-strain beim Referenz-PEEP von 15 cmH2O, wodurch eine Normierung erzielt wurde, die es ermöglichte, vergleichbare relative strain-Bilder zu erstellen, die die Grundlage für die Berechnung numerischer Werte des totalen strains für die jeweilige PEEP-Stufe bildete. Hierzu wurden die jeweiligen Pixelwerte aufsummiert und bezüglich der Anzahl aller Pixel in der Lungen ROI eines jeden Schweines normiert. Schließlich wurden aus den zuvor genannten strain-Bildern EIT-Bilder generiert, die den mittleren relativen strain aller Probanden auf dem jeweiligen PEEP-Niveau repräsentierten.

Erfindungsgemäß wird die Mess- und Regelungstechnik des Beatmungsgerätes mit der Elektrischen Impedanz Tomographie EIT) vernetzt. Dadurch kann die Funktion der Lunge kontinuierlich bildgebend dargestellt werden. Die Messwerte des Beatmungsgerätes werden mit den Ergebnissen der elektrischen Impedanztomographie kombiniert. So ermöglicht es diese Technologie, die vielfältigsten klinischen Fragestellungen zu bewerten und die Therapie entsprechend auszurichten.

**Patentansprüche**

1. System (10) zur Echtzeit-Bestimmung einer lokalen Beanspruchung einer Lunge während einer künstlichen Beatmung (1) mit einem Beatmungsgerät, wobei das Beatmungsgerät Teil des Systems (10) ist; wobei eine Einrichtung zur Elektro-Impedanz-Tomographie (EIT) (3) vorgesehen ist, die dazu eingerichtet ist, eine elektrische Impedanzverteilung entlang wenigstens eines zweidimensionalen Schnitts durch einen menschlichen Thorax zu erfassen; wobei eine Einrichtung zur Zuordnung (33) der erfassten elektrischen Impedanzverteilung vorgesehen ist, die dazu eingerichtet ist, die erfasste elektrische Impedanzverteilung zu verschiedenen Zeitpunkten während der künstlichen Beatmung in eine Vielzahl von EIT-Pixeln zu unterteilen und einen spezifischen Wert der elektrischen Impedanz zu einem spezifischen Zeitpunkt einem spezifischen EIT-Pixel zuzuweisen, wobei eine Einrichtung zur Bestimmung eines lokalen Beanspruchungswertes (333) vorgesehen ist, die dazu eingerichtet ist; zumindest eine end-inspiratorische elektrische Impedanz ($Z_{INSP}$) des spezifischen EIT-Pixels sowie eine end-exspiratorische elektrische Impedanz ($Z_{EXSP}$) des spezifischen EIT-Pixels zu bestimmen und einen lokalen Tidalvolumenreferenzwert ($\Delta Z$) des spezifischen EIT-Pixels als Differenz aus der end-inspiratorischen elektrischen Impedanz ($Z_{INSP}$) und der end-exspiratorischen elektrischen Impedanz ($Z_{EXSP}$) durch Subtraktion der end-exspiratorischen elektrischen Impedanz ($Z_{EXSP}$) von der end-inspiratorischen elektrischen Impedanz ($Z_{INSP}$) zu bilden, **dadurch gekennzeichnet, dass** die Einrichtung zur Bestimmung eines lokalen Beanspruchungswertes (333) dazu eingerichtet ist; einen vorläufigen Beanspruchungswert ($STR_{VORL}$) des spezifischen EIT-Pixels durch Dividieren des lokalen Tidalvolumenreferenzwertes ($\Delta Z$) durch die end-exspiratorische elektrische Impedanz ($Z_{EXSP}$) zu bilden und einen relativen Beanspruchungswert ($STR_{RELATIV}$) des spezifischen EIT-Pixels durch Normierung des vorläufigen Beanspruchungswert ($STR_{VORL}$) auf einen Referenz-Beanspruchungswert ($STR_{REF}$) zu bilden.

2. System nach zumindest einem der vorhergehenden Ansprüche **dadurch gekennzeichnet, dass** die Einrichtung dazu eingerichtet ist, den vorläufigen Beanspruchungswert ($STR_{VORL}$) auf einen Quotienten aus dem lokalen Tidalvolumenreferenzwert ($\Delta Z$) und der end-exspiratorischen elektrischen Impedanz ($Z_{EXSP}$) des spezifischen EIT-Pixels bei einem vorgegebenen positiven endexspiratorischen Druck (PEEP) zu normieren.

3. System nach zumindest einem der vorhergehenden Ansprüche **dadurch gekennzeichnet, dass** eine Einrichtung zur Elektro-Impedanz-Tomographie (EIT) vorgesehen ist, die dazu eingerichtet ist, eine elektrische Impedanzverteilung entlang wenigstens eines zweidimensionalen Schnitts durch einen menschlichen Thorax zu erfassen, wobei eine Einrichtung zur Zuordnung der erfassten elektrischen Impedanzverteilung vorgesehen ist, die dazu eingerichtet ist, die erfasste elektrische Impedanzverteilung zu verschiedenen Zeitpunkten während der künstlichen Beatmung in eine Vielzahl von EIT-Pixeln zu unterteilen und einen spezifischen Wert der elektrischen Impedanz zu einem spezifischen Zeitpunkt einem spezifischen EIT-Pixel zuzuweisen; wobei eine Einrichtung zur Bestimmung eines lokalen Beanspruchungswertes vorgesehen ist, die dazu eingerichtet ist;

   zumindest eine end-inspiratorische elektrische Impedanz ($Z_{INSP}$) des spezifischen EIT-Pixels sowie eine end-exspiratorische elektrische Impedanz ($Z_{EXSP}$) des spezifischen EIT-Pixels zu bestimmen,
   einen lokalen Tidalvolumenreferenzwert ($\Delta Z$) des spezifischen EIT-Pixels als Differenz aus der end-inspiratorischen elektrischen Impedanz ($Z_{INSP}$) und der end-exspiratorischen elektrischen Impedanz ($Z_{EXSP}$) zu bilden, insbesondere durch Subtraktion der end-exspiratorischen elektrischen Impedanz ($Z_{EXSP}$) von der end-inspiratorischen elektrischen Impedanz ($Z_{INSP}$) zu bilden, und
   einen vorläufigen Beanspruchungswert ($STR_{VORL}$) des spezifischen EIT-Pixels durch Dividieren des lokalen Tidalvolumenreferenzwertes ($\Delta Z$) durch die end-exspiratorische elektrische Impedanz ($Z_{EXSP}$) zu bilden;
   und wobei die Einrichtung zur Bestimmung eines lokalen Beanspruchungswertes ferner dazu eingerichtet ist,
   einen relativen Beanspruchungswert ($STR_{RELATIV}$) des spezifischen EIT-Pixels durch Normierung des vorläufigen Beanspruchungswert ($STR_{VORL}$) auf einen Referenz-Beanspruchungswert ($STR_{REF}$) zu bilden.

4. System zur automatisierten Einstellung eines durch ein Beatmungsgerät vorgegebenen Wertes, insbesondere eines Drucks, vorzugsweise eines positiven endexspiratorischen Drucks (PEEP), umfassend ein System nach einem der vorhergehenden Ansprüche, wobei eine Regelungseinrichtung vorgesehen ist, die dazu eingerichtet ist, den durch das Beatmungsgerät vorgegebenen Wert auf Grundlage der gebildeten relativen Beanspruchungswerte ($STR_{RELATIV}$) anzupassen.

5. Beatmungsgerät welches eingerichtet und ausgebildet ist zur Verwendung in einem System nach zumindest einem der vorhergehenden Ansprüche 1 bis 4, wobei das Beatmungsgerät eine Steuereinrichtung (21) umfasst, wobei das Beatmungsgerät wenigstens eine steuerbare Atemgasquelle (100) und zumindest eine Sensoreinrichtung (2) zur Bestimmung von Druck und/oder Fluss des Atemgases umfasst, wobei die Steuereinrichtung (21) die Atemgasquelle zur Vorgabe eines ersten vorbestimmten Beatmungsmusters (45) ansteuert, wobei das Beatmungsgerät zudem wenigstens zwei weitere Sensoreinrichtungen (3, 30, 40) umfasst, die mit der Steuereinrichtung (21) des Beatmungsgerätes (1) kommunizieren, wobei die Einrichtung zur Elektro-Impedanz-Tomographie (EIT) (3) dazu eingerichtet ist, eine elektrische Impedanzverteilung entlang wenigstens eines zweidimensionalen Schnitts durch einen menschlichen Thorax zu erfassen wobei die Steuereinrichtung (21) dazu eingerichtet ist die Sensormesswerte der Einrichtung zur Elektro-Impedanz-Tomographie (EIT) (3) auszuwerten, wobei eine Einrichtung zur Zuordnung (33) der erfassten elektrischen Impedanzverteilung im Beatmungsgerät vorgesehen ist, die dazu eingerichtet ist, die erfasste elektrische Impedanzverteilung zu verschiedenen Zeitpunkten während der künstlichen Beatmung in eine Vielzahl von EIT-Pixeln zu unterteilen und einen spezifischen Wert der elektrischen Impedanz zu einem spezifischen Zeitpunkt einem spezifischen EIT-Pixel zuzuweisen, wobei im Beatmungsgerät eine Einrichtung zur Bestimmung eines lokalen Beanspruchungswertes (333) vorgesehen ist, die dazu eingerichtet ist; zumindest eine end-inspiratorische elektrische Impedanz ($Z_{INSP}$) des spezifischen EIT-Pixels sowie eine end-exspiratorische elektrische Impedanz ($Z_{EXSP}$) des spezifischen EIT-Pixels zu bestimmen und einen lokalen Tidalvolumenreferenzwert ($\Delta Z$) des spezifischen EIT-Pixels als Differenz aus der end-inspiratorischen elektrischen Impedanz ($Z_{INSP}$) und der end-exspiratorischen elektrischen Impedanz ($Z_{EXSP}$) durch Subtraktion der end-exspiratorischen elektrischen Impedanz ($Z_{EXSP}$) von der end-inspiratorischen elektrischen Impedanz ($Z_{INSP}$) zu bilden, **dadurch gekennzeichnet, dass** die Einrichtung zur Bestimmung eines lokalen Beanspruchungswertes (333) dazu eingerichtet ist; einen vorläufigen Beanspruchungswert ($STR_{VORL}$) des spezifischen EIT-Pixels durch Dividieren des lokalen Tidalvolumenreferenzwertes ($\Delta Z$) durch die end-exspiratorische elektrische Impedanz ($Z_{EXSP}$) zu bilden und einen relativen Beanspruchungswert ($STR_{RELATIV}$) des spezifischen EIT-Pixels durch Normierung des vorläufigen Beanspruchungswert ($STR_{VORL}$) auf einen Referenz-Beanspruchungswert ($STR_{REF}$) zu bilden.

6. Beatmungsgerät (1), nach Anspruch 5, welches eingerichtet und ausgebildet ist, die Beanspruchung der gesamten Lungen, die die atemzugsbedingte Verformung des Lungengewebes hervorruft, unter Nutzung der EIT-Informationen auf Pixelebene durchzuführen.

7. Beatmungsgerät (1), nach zumindest einem der vorhergehenden Ansprüche, welches eingerichtet und ausgebildet ist, eine Bestimmung der lokalen Beanspruchung (strain) der Lunge, unter Nutzung der EIT-Informationen auf Pixelebene, durchzuführen, indem das Tidalvolumen eines jeden Pixels aus seiner jeweiligen Impedanzänderung (delta Z) während des Atemzyklus bestimmt und dieses sodann ins Verhältnis zu seinem jeweiligen end-exspiratorischen Volumen, das seinem Impedanzwert zum Ende der Exspiration (EELI) entspricht, gesetzt wird.

8. Beatmungsgerät (1), nach zumindest einem der vorhergehenden Ansprüche, wobei die Sensoreinrichtung zur Erzeugung von elektrischen Potenzialen und/oder zur nichtinvasiven Bestimmung der elektrischen Impedanz (EI) eines Körperabschnitts und zur Ermittlung und Übertragung der Sensormesswerte an die Steuereinheit ausgebildet ist, wobei die Steuereinrichtung (21) die Sensormesswerte der EI-Sensoreinrichtung (3) auswertet, zur Bestimmung der momentanen (44) Beanspruchung der Lunge während der Beatmung mit einem ersten Beatmungsmuster (45) und wobei die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wobei die Steuereinrichtung (21) die Sensormesswerte der EI-Sensoreinrichtung (3) auswertet zur Bestimmung des resultierenden (54) Beanspruchung der Lunge während der Beatmung mit dem zweiten Beatmungsmuster (55).

9. Beatmungsgerät (1), nach zumindest einem der vorhergehenden Ansprüche, wobei die Steuereinrichtung zumindest einen Druck beispielsweise den PEEP oder ein Beatmungsmuster so anpasst, dass die Beanspruchung reduziert wird.

**Claims**

1. A system (10) for real-time determination of a local strain of a lung during artificial ventilation (1) using a ventilator, wherein the ventilator is part of the system (10), wherein the system (10) comprises a device for electrical impedance tomography (EIT) (3), which device is configured to capture an electrical impedance distribution along at least one two-dimensional section through a human thorax, and further comprises a device for assigning (33) the captured electrical impedance distribution, which device is configured to divide the captured electrical impedance distribution at different times during the artificial ventilation into a multiplicity of EIT pixels and to assign a specific value of the electrical impedance at a specific time to a specific EIT pixel, wherein the system further comprises a device for determining a local strain value (333), which device is configured to determine at least one end-inspiratory electrical impedance ($Z_{INSP}$) of the specific EIT pixel and one end-expiratory electrical impedance ($Z_{EXSP}$) of the specific EIT pixel and to form a local tidal volume reference value ($\Delta Z$) of the specific EIT pixel as a difference between the end-inspiratory electrical impedance ($Z_{INSP}$) and the end-expiratory electrical impedance ($Z_{EXSP}$) by subtracting the end-expiratory electrical impedance ($Z_{EXSP}$) from the end-inspiratory electrical impedance ($Z_{INSP}$), **characterized in that** the device for determining a local strain value (333) is configured to form a preliminary strain value ($STR_{VORL}$) of the specific EIT pixel by dividing the local tidal volume reference value ($\Delta Z$) by the end-expiratory electrical impedance ($Z_{EXSP}$) and to form a relative strain value ($STR_{RELATIV}$) of the specific EIT pixel by normalizing the preliminary strain value ($STR_{VORL}$) to a reference strain value ($STR_{REF}$).

2. The system of at least one of the preceding claims, **characterized in that** the device is configured to normalize the preliminary strain value ($STR_{VORL}$) to a quotient of the local tidal volume reference value ($\Delta Z$) and the end-expiratory electrical impedance ($Z_{EXSP}$) of the specific EIT pixel at a specified positive end-expiratory pressure (PEEP).

3. The system of at least one of the preceding claims, **characterized in that** the system comprises a device for electrical impedance tomography (EIT), which device is configured to capture an electrical impedance distribution along at least one two-dimensional section through a human thorax, and further comprises a device for assigning the captured electrical impedance distribution, which device is configured to divide the captured electrical impedance distribution at different times during the artificial ventilation into a multiplicity of EIT pixels and to assign a specific value of the electrical impedance at a specific time to a specific EIT pixel;

   wherein the system further comprises a device for determining a local strain value, which device is configured to determine at least one end-inspiratory electrical impedance ($Z_{INSP}$) of the specific EIT pixel and an end-expiratory electrical impedance ($Z_{EXSP}$) of the specific EIT pixel,
   to form a local tidal volume reference value ($\Delta Z$) of the specific EIT pixel as a difference between the end-inspiratory electrical impedance ($Z_{INSP}$) and the end-expiratory electrical impedance ($Z_{EXSP}$), in particular by subtracting the end-expiratory electrical impedance ($Z_{EXSP}$) from the end-inspiratory electrical impedance ($Z_{INSP}$), and
   to form a preliminary strain value ($STR_{VORL}$) of the specific EIT pixel by dividing the local tidal volume reference value ($\Delta Z$) by the end-expiratory electrical impedance ($Z_{EXSP}$);
   and wherein the device for determining a local strain value is further configured to form a relative strain value ($STR_{RELATIV}$) of the specific EIT pixel by normalizing the preliminary strain value ($STR_{VORL}$) to a reference strain value ($STR_{REF}$).

4. A system for automatically setting a value specified by a ventilator, in particular of a pressure, preferably of a positive end-expiratory pressure (PEEP), wherein the system comprises a system of one of the preceding claims and a closed-loop control device that is configured to adapt the value specified by the ventilator on the basis of the relative strain values ($STR_{RELATIV}$) formed.

5. A ventilator configured and embodied for use in a system of at least one of claims 1 to 4, wherein the ventilator comprises a control device (21), wherein the ventilator comprises at least one controllable respiratory gas source (100) and at least one sensor device (2) for determining pressure and/or flow of the respiratory gas, wherein the control device (21) drives the respiratory gas source for specifying a first predetermined ventilation pattern (45), wherein the ventilator further comprises at least two further sensor devices (3, 30, 40) that communicate with the control device (21) of the ventilator (1), wherein the device for electrical impedance tomography (EIT) (3) is configured to capture an electrical impedance distribution along at least one two-dimensional section through a human thorax, wherein the control device (21) is configured to evaluate the sensor measured values of the device for electrical impedance tomography (EIT) (3), wherein the ventilator comprises a device for assigning (33) the captured electrical impedance distribution, which device is configured to divide the captured electrical impedance distribution at different times during the artificial ventilation into a multiplicity of EIT pixels and to assign a specific value of the electrical impedance at a specific time to a specific EIT pixel, wherein the ventilator comprises a device for determining a local strain value (333), which device is configured to determine at least one end-inspiratory electrical impedance ($Z_{INSP}$) of the specific EIT pixel and one end-expiratory electrical impedance ($Z_{EXSP}$) of the specific EIT pixel and to form a local tidal volume reference value ($\Delta Z$) of the specific EIT pixel as a difference between the end-inspiratory electrical impedance ($Z_{INSP}$) and the end-expiratory electrical impedance ($Z_{EXSP}$) by subtracting the end-expiratory electrical impedance ($Z_{EXSP}$) from the end-inspiratory electrical impedance ($Z_{INSP}$), **characterized in that** the device for determining a local strain value (333) is configured to form a preliminary strain value ($STR_{VORL}$) of the specific EIT pixel by dividing the local tidal volume reference value ($\Delta Z$) by the end-expiratory electrical impedance ($Z_{EXSP}$) and to form a relative strain value ($STR_{RELATIV}$) of the specific EIT pixel by normalizing the preliminary strain value ($STR_{VORL}$) to a reference strain value ($STR_{REF}$).

6.  The ventilator (1) of claim 5, wherein the ventilator is configured and embodied to perform the global strain of the lungs, which causes the respiration-dependent deformation of the lung tissue, using the EIT information at a pixel level.

7.  The ventilator (1) of at least one of the preceding claims, wherein the ventilator is configured and embodied to perform a determination of the local strain of the lung using the EIT information at a pixel level, by virtue of the tidal volume of each pixel being determined from its respective change in impedance (delta Z) during the respiratory cycle and said tidal volume thereupon being related to its respective end-expiratory volume, which corresponds to its impedance value at the end of expiration (EELI).

8.  The ventilator (1) of at least one of the preceding claims, wherein the sensor device is configured to generate electrical potentials and/or to non-invasively determine the electrical impedance (EI) of a body section and to determine and transfer the sensor measured values to the control unit, wherein the control device (21) evaluates the sensor measured values of the EI sensor device (3) for the purposes of determining the current (44) strain of the lung during the ventilation with a first ventilation pattern (45) and wherein the control device (21) drives the respiratory gas source for specifying a second ventilation pattern (55), which differs from the first ventilation pattern (45) in terms of pressure and/or flow and/or volume and/or frequency, wherein the control device (21) evaluates the sensor measured values of the EI sensor device (3) for the purposes of determining a resultant (54) strain of the lung during the ventilation with the second ventilation pattern (55).

9.  The ventilator (1) of at least one of the preceding claims, wherein the control device adapts at least one pressure, for example, the PEEP, or a ventilation pattern in such a way that the strain is reduced.

**Revendications**

1.  Système (10) pour la détermination en temps réel d'une contrainte locale d'un poumon pendant une respiration artificielle (1) avec un appareil respiratoire, l'appareil respiratoire faisant partie du système (10) ; un dispositif pour la tomographie d'impédance électrique (EIT) (3) étant prévu, lequel est agencé pour détecter une répartition d'impédance électrique le long d'au moins une section bidimensionnelle à travers un thorax humain ; un dispositif pour l'attribution (33) de la répartition d'impédance électrique détectée étant prévu, lequel est agencé pour subdiviser la répartition d'impédance électrique détectée à différents instants pendant la respiration artificielle en une multiplicité de pixels EIT et pour attribuer à un pixel EIT spécifique une valeur spécifique de l'impédance électrique à un instant spécifique, un dispositif pour la détermination d'une valeur de contrainte locale (333) étant prévu, lequel est agencé pour déterminer au moins une impédance électrique en fin d'inspiration ($Z_{INSP}$) du pixel EIT spécifique ainsi qu'une impédance électrique en fin d'expiration ($Z_{EXSP}$) du pixel EIT spécifique et pour former une valeur de référence de volume tidal ($\Delta Z$) du pixel EIT spécifique comme différence entre l'impédance électrique en fin d'inspiration ($Z_{INSP}$) et l'impédance électrique en fin d'expiration ($Z_{EXSP}$) par soustraction de l'impédance électrique en fin d'expiration ($Z_{EXSP}$) de l'impédance électrique en fin d'inspiration ($Z_{INSP}$), **caractérisé en ce que** le dispositif pour la détermination d'une valeur de contrainte locale (333) est agencé pour former une valeur de contrainte provisoire ($STR_{VORL}$) du pixel EIT spécifique par division de la valeur de référence de volume tidal ($\Delta Z$) par l'impédance électrique en fin d'expiration ($Z_{EXSP}$) et pour former une valeur de contrainte relative ($STR_{RELATIV}$) du pixel EIT spécifique par normalisation de la valeur de contrainte provisoire ($STR_{VORL}$) sur une valeur de contrainte de référence ($STR_{REF}$).

2.  Système selon au moins l'une des revendications précédentes, **caractérisé en ce que** le dispositif est agencé pour normaliser la valeur de contrainte provisoire ($STR_{VORL}$) sur un quotient entre la valeur de référence de volume tidal locale ($\Delta Z$) et l'impédance électrique en fin d'expiration ($Z_{EXSP}$) du pixel EIT spécifique à une pression en fin d'expiration positive spécifiée (PEEP).

3.  Système selon au moins l'une des revendications précédentes, **caractérisé en ce qu'**un dispositif est prévu pour la tomographie d'impédance électrique (EIT), lequel est agencé pour détecter une répartition d'impédance électrique le long d'au moins une section bidimensionnelle à travers un thorax humain, un dispositif pour l'attribution de la répartition d'impédance électrique détectée étant prévu, lequel est agencé pour subdiviser la répartition d'impédance électrique détectée à différents instants pendant la respiration artificielle en une multiplicité de pixels EIT et pour attribuer à un pixel EIT spécifique une valeur spécifique de l'impédance électrique à un instant spécifique ; un dispositif pour la détermination d'une valeur de contrainte locale étant prévu, lequel est agencé

pour déterminer au moins une impédance électrique en fin d'inspiration ($Z_{INSP}$) du pixel EIT spécifique ainsi qu'une impédance électrique en fin d'expiration ($Z_{EXSP}$) du pixel EIT spécifique,
pour former une valeur de référence de volume tidal locale ($\Delta Z$) du pixel EIT spécifique comme différence entre l'impédance électrique en fin d'inspiration ($Z_{INSP}$) et l'impédance électrique en fin d'expiration ($Z_{EXSP}$), en particulier par soustraction de l'impédance électrique en fin d'expiration ($Z_{EXSP}$) de l'impédance électrique en fin d'inspiration ($Z_{INSP}$), et
pour former une valeur de contrainte provisoire ($STR_{VORL}$) du pixel EIT spécifique par division de la valeur de référence de volume tidal locale ($\Delta Z$) par l'impédance électrique en fin d'expiration ($Z_{EXSP}$) ; et
le dispositif pour la détermination de la valeur de contrainte locale étant en outre agencé pour former une valeur de contrainte relative ($STR_{RELATIV}$) du pixel EIT spécifique par la normalisation de la valeur de contrainte provisoire ($STR_{VORL}$) sur une valeur de contrainte de référence ($STR_{REF}$).

4. Système pour le réglage automatique d'une valeur spécifiée par un appareil respiratoire, en particulier d'une pression, de préférence d'une pression en fin d'expiration positive (PEEP), comprenant un système selon l'une des revendications précédentes, un dispositif de réglage étant prévu, lequel est agencé pour ajuster la valeur spécifiée par l'appareil respiratoire sur la base des valeurs de contrainte relatives ($STR_{RELATIV}$) formées.

5. Appareil respiratoire, lequel est agencé et conçu pour l'utilisation dans un système selon au moins l'une des revendications 1 à 4, l'appareil respiratoire comprenant un dispositif de commande (21), l'appareil respiratoire comprenant au moins une source de gaz de respiration (100) pouvant être commandée et au moins un dispositif de capteur (2) pour la détermination de la pression et/ou de l'écoulement du gaz de respiration, le dispositif de commande (21) actionnant la source de gaz de respiration pour la spsfkc d'un premier modèle respiratoire prédéterminé (45), l'appareil respiratoire comprenant en outre au moins deux dispositifs de capteurs supplémentaires (3, 30, 40), lesquels communiquent avec le dispositif de commande (21) de l'appareil respiratoire (1),
le dispositif pour la tomographie d'impédance électrique (EIT) (3) étant agencé pour détecter une répartition d'impédance électrique le long d'au moins une section bidimensionnelle à travers un thorax humain, le dispositif de commande (21) étant agencé pour évaluer les valeurs de mesure de capteur du dispositif pour la tomographie d'impédance électrique (EIT) (3), un dispositif pour l'attribution (33) de la répartition d'impédance électrique détectée étant prévu dans l'appareil respiratoire, lequel est agencé pour subdiviser la répartition d'impédance électrique détectée à différents instants pendant la respiration artificielle en une multiplicité de pixels EIT et pour attribuer à un pixel EIT spécifique une valeur spécifique de l'impédance électrique à un instant spécifique, un dispositif pour la détermination d'une valeur de contrainte locale (333) étant prévu dans l'appareil respiratoire, lequel est agencé pour déterminer au moins une impédance électrique en fin d'inspiration ($Z_{INSP}$) du pixel EIT spécifique ainsi qu'une impédance électrique en fin d'expiration ($Z_{EXSP}$) du pixel EIT spécifique, et pour former une valeur de référence de volume tidal locale ($\Delta Z$) du pixel EIT spécifique comme différence entre l'impédance électrique en fin d'inspiration ($Z_{INSP}$) et l'impédance électrique en fin d'expiration ($Z_{EXSP}$) par soustraction de l'impédance électrique en fin d'expiration ($Z_{EXSP}$) de l'impédance électrique en fin d'inspiration ($Z_{INSP}$), **caractérisé en ce que** le dispositif pour la détermination d'une valeur de contrainte locale (333) est agencé pour former une valeur de contrainte provisoire ($STR_{VORL}$) du pixel EIT spécifique par division de la valeur de référence de volume tidal locale ($\Delta Z$) par l'impédance électrique en fin d'expiration ($Z_{EXSP}$), et pour former une valeur de contrainte relative ($STR_{RELATIV}$) du pixel EIT spécifique par la normalisation de la valeur de contrainte provisoire ($STR_{VORL}$) sur une valeur de contrainte de référence ($STR_{REF}$).

6. Appareil respiratoire (1) selon la revendication 5, lequel est agencé et formé pour réaliser la contrainte de l'ensemble des poumons, laquelle est entraînée par la déformation du tissu pulmonaire due à une respiration, avec utilisation des informations EIT au niveau des pixels.

7. Appareil respiratoire (1) selon au moins l'une des revendications précédentes, lequel est agencé et formé pour réaliser une détermination de la contrainte locale (strain) du poumon, avec utilisation des informations EIT au niveau des pixels, le volume tidal de chaque pixel étant déterminé pendant le cycle respiratoire à partir de sa modification d'impédance respective (delta Z), et en ce que ce volume est ensuite mis en rapport avec son volume respectif en fin d'expiration, lequel correspond à sa valeur d'impédance en fin d'expiration (EELI).

**8.** Appareil respiratoire (1) selon au moins l'une des revendications précédentes, le dispositif de capteur étant conçu pour la génération de potentiels électriques et/ou pour la détermination non invasive de l'impédance électrique (EI) d'une partie du corps et pour la détermination et le transfert des valeurs de capteur à l'unité de commande, le dispositif de commande (21) évaluant les valeurs de mesure de capteur du dispositif de capteur EI (3), pour la détermination de la contrainte actuelle (44) du poumon pendant la respiration avec un premier modèle de respiration (45) et le dispositif de commande (21) actionnant la source de gaz de respiration pour la spécification d'un deuxième modèle respiratoire (55), lequel est différent du premier modèle respiratoire (45) en ce qui concerne la pression et/ou l'écoulement et/ou le volume et/ou la fréquence, le dispositif de commande (21) évaluant les valeurs de mesure de capteur du dispositif de capteur EI (3) pour la détermination de la contrainte résultante (54) du poumon pendant la respiration avec le deuxième modèle respiratoire (55).

**9.** Appareil respiratoire (1) selon au moins l'une des revendications précédentes, le dispositif de commande ajustant au moins une pression, par exemple la PEEP, ou un modèle respiratoire de telle sorte que la contrainte est réduite.

Fig. 1

Fig. 2

EP 3 725 222 B1

Ventilation

Regionale Überdehnung bei niedrigem Druck

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013203177 A1

**In der Beschreibung aufgeführte Nicht-Patentliteratur**

- *Crit Care Med,* 2007, vol. 35 (1), 214-221
- *Intensive Care Med*